# EUROPÄISCHE PATENTSCHRIFT

(11) **EP 3 036 519 B1**
(45) Veröffentlichungstag und Bekanntmachung des Hinweises auf die Patenterteilung: **27.10.2021**
(21) Anmeldenummer: 14747840.8
(22) Anmeldetag: 31.07.2014
(51) Int. Cl.: G01N 1/22

(54) **GASENTNAHMESONDE UND VERFAHREN ZUM BETREIBEN EINER GASENTNAHMESONDE**
GAS-SAMPLING PROBE AND METHOD FOR OPERATING A GAS-SAMPLING PROBE
SONDE DE PRÉLÈVEMENT DE GAZ ET PROCÉDÉ D'UTILISATION D'UNE SONDE DE PRÉLÈVEMENT DE GAZ

(30) Priorität: 19.08.2013 DE 102013108926
(43) Veröffentlichungstag der Anmeldung: 29.06.2016
(73) Patentinhaber: thyssenkrupp Industrial Solutions AG, 45143 Essen (DE)
(72) Erfinder: KÖNNING, Ludwig, 59227 Ahlen - Vorhelm (DE); STREFFING, Michael, 59510 Lippetal-Hovestadt (DE); BREDEMEIER, Heinz, 48336 Sassenberg (DE); LEUER, Alfons, 59302 Oelde (DE)
(74) Vertreter: thyssenkrupp Intellectual Property GmbH
(86) Internationale Anmeldenummer: PCT/EP2014/002100
(87) Internationale Veröffentlichungsnummer: WO 2015/024625

(56) Entgegenhaltungen:
- AT-U1- 9 667
- DE-A1- 3 545 491
- DE-U1- 29 608 694
- FR-A1- 2 715 732

## Beschreibung

Die Erfindung betrifft eine Gasentnahmesonde und ein Verfahren zum Betreiben einer Gasentnahmesonde, wobei ein zu analysierendes Gas im Bereich eines vorderen Endes eines Gasentnahmerohrs aus einem Prozessraum entnommen und im Gasentnahmerohr bis zu einem hinteren Ende geführt und dabei gekühlt wird.

Aus der DE 103 15 996 A1 ist eine Sonde zu Entnahme einer Gasprobe aus einem heißen Reaktionsraum bekannt, wobei ein durch einen Außenmantel begrenzter Ringraum mit Kühlwasser durchströmt wird. Da der Siedepunkt des Wassers nicht erreicht werden darf, liegt die maximale Rücklauftemperatur bei ca. 90°C. Bei diesen niedrigen Temperaturen können aber Taupunktunterschreitungen innerhalb der Gasentnahmesonde nicht sicher ausgeschlossen werden. Deshalb wird bei einigen Herstellern das Gasentnahmerohr zusätzlich elektrisch beheizt, um so die Taupunktunterschreitungen im Messgas zu verhindern. In der DE 103 15 996 A1 wird die Taupunktunterschreitung dadurch verhindert, dass das Gasentnahmerohr mit einem evakuierbaren Hohlraum umgeben ist. Die starke Wasserkühlung hat aber auch den weiteren Nachteil, dass der äußere Teil der Gasentnahmesonde unnötig stark abgekühlt wird, wobei eine zu kalte Spitze der Gasentnahmesonde im heißen Prozessgas zu Ansatzbildung neigen kann.

Des Weiteren sind ölgekühlte Gasentnahmesonden bekannt, die zur Kühlung ein Wärmeträgeröl einsetzen. Der Unterschied zum Wasserkreislauf besteht darin, dass das Wärmeträgeröl in höheren Temperaturbereichen betrieben werden kann. Dadurch kann eine zusätzliche Heizung des Gasentnahmerohres entfallen. Problematisch ist das Wärmeträgeröl aber hinsichtlich Leckagen zu beurteilen, da hier eine Brandgefährdung nicht auszuschließen ist.

Aus der DE 103 54 188 A1 ist ferner eine Hochtemperaturentnahmesonde bekannt, bei der ein Gasentnahmerohr von einem äußeren Rohr umgeben ist und zwischen diesen beiden Rohren unter Druck stehende Kühlluft geführt wird. Dabei wurde darauf geachtet, dass das entnommene, zu analysierende Gas entlang des Gasentnahmerohres nicht unter 250° abgekühlt wird und so Kondensationen vermieden werden.

Die DE 35 45 491 A1 offenbart eine Sondenanordnung für die Gasentnahme aus einem Drehrohr-Zementofen, die mittels Kühlflüssigkeit gekühlt wird.

In der AT 9 667 U1 wird eine Einrichtung an Gasanalysegeräten zur Messung der Gaskonzentration beschrieben, wobei eine Leitung für das zu messende Gas wenigstens teilweise von einem im Abstand zu ihr befindlichen Mantel umgeben ist und durch den zwischen der Leitung und dem Mantel bestehenden Raum im Gegenstrom Luft geleitet wird, die zur Einhaltung einer vorgegebenen Temperatur des zu messenden Gases beheizt oder gekühlt wird.

Der Erfindung liegt die Aufgabe zugrunde, eine neues Konzept zum Betreiben einer Gasentnahmesonde anzugeben, mit dem eine ausreichende Kühlung des vorderen Endes des Gasentnahmerohres gewährleistet ist und eine Unterschreitung der Taupunkttemperatur der im zu analysierenden Gas enthaltenden Komponenten zuverlässig vermieden wird.

Erfindungsgemäß wird diese Aufgabe durch die Merkmale der Ansprüche 1 und 16 gelöst. Weitere Ausgestaltungen sind Gegenstand der Unteransprüche.

Beim erfindungsgemäßen Verfahren zum Betreiben einer Gasentnahmesonde wird ein zu analysierendes Gas im Bereich eines vorderen Endes eines Gasentnahmerohrs aus einem Prozessraum entnommen und im Gasentnahmerohr bis zu einem hinteren Ende geführt und dabei gekühlt, indem zwischen dem Gasentnahmerohr und wenigstens einem das Gasentnahmerohr umgebenden Außenmantel Kühlluft geführt wird, wobei die Kühlluft am hinteren Ende des Gasentnahmerohres zu- und abgeführt wird und die Temperatur des zu analysierenden Gases im Bereich des vorderen Endes des Gasentnahmerohrs höher als die Temperatur der zugeführten Kühlluft ist, wobei die Gasentnahmesonde nach außen abstrahlt und die Temperatur der zugeführten Kühlluft höher als die Temperatur der abgeführten Kühlluft ist.

Die erfindungsgemäße Gasentnahmeeinrichtung zur Durchführung des obigen Verfahrens weist ein Gasentnahmerohr auf, um ein zu analysierendes Gas im Bereich eines vorderen Endes zu entnehmen und im Gasentnahmerohr bis zu einem hinteren Ende zu führen, wobei das Gasentnahmerohr von wenigstens einem Außenmantel umgeben ist, sodass eine sich über die Länge des Gasentnahmerohres erstreckende Kühlzone ausgebildet ist, die im Bereich des hinteren Endes der Gasentnahmesonde eine Kühlluftzuführöffnung zum Zuführen von Kühlluft und eine Kühlluftabführöffnung zum Abführen von Kühlluft aufweist, wobei die Kühlluftabführöffnung und die Kühlluftzuführöffnung zur Ausbildung eines geschlossenen Kreislaufs miteinander verbunden sind und die Gasentnahmesonde nach außen abstrahlt. Zwischen der Kühlluftabführöffnung und der Kühlluftzuführöffnung ist außerdem ein Lufterhitzer zum Erhöhen der Temperatur der abgeführten Kühlluft vorgesehen.

Das erfindungsgemäße Konzept bei dem die Temperatur der zugeführten Kühlluft höher als die Temperatur der abgeführten Kühlluft ist, beruht auf der Ausnutzung der Tatsache, dass dem Prozess nur niedrige Wärmemengen entnommen werden und den Wärmeverlusten der Gasentnahmesonde nach außen. Die durch das Gasentnahmerohr geführte Kühlluft bewirkt zum einen, dass die Temperatur des vorderen Endes der Gasentnahmesonde reduziert wird, während das Gasentnahmerohr im hinteren Bereich erwärmt wird. Gleichzeitig wird das entnommene, zu analysierende Gas vom vorderen bis zum hinteren Ende abgekühlt. Es findet somit eine Vergleichmäßigung der Temperatur des Gasentnahmerohrs über seine gesamte Länge statt.

Durch die Kühlung mit Luft kann im Vergleich zur Wasserkühlung die Temperatur des vorderen Endes des Gasentnahmerohres gezielt angehoben werden, um die Gefahr von äußeren Anbackungen zu vermindern. Dies ist dadurch bedingt, dass bei der Wasserkühlung die Oberflächentemperatur die Gasentnahmesonde deutlich geringer ist, da der Wärmeübergangskoeffizient zwischen Wasser und Sondenwand deutlich größer ist als zwischen Luft und Sondenwand. Zusätzlich kann Wasser aufgrund der höheren Wärmekapazität mehr Wärme abführen. Des Weiteren werden bei der erfindungsgemäßen Lösung Anbackungen bzw. Kondensationen im Gasentnahmerohr durch die höheren Betriebstemperaturen ebenfalls minimiert.

Weitere Ausgestaltungen der Erfindung sind Gegenstand der Unteransprüche.

Die Gasentnahmesonde ist vorzugsweise so am Prozessraum angeordnet, dass sie in einem vorderen, dem Prozessraum zugewandten Bereich Wärme von außen aufnimmt und in einem hinteren Bereich Wärme nach außen abstrahlt, wobei in einer Gesamtwärmebilanz die Gasentnahmesonde mehr Wärme abstrahlt als aufnimmt.

Der Differenzbetrag der aufgenommenen und abgestrahlten Wärme der Gasentnahmesonde entspricht der Summe der Kühlwärme des zu analysierendes Gases und der Kühlluft.

Gemäß einer bevorzugten Ausgestaltung der Erfindung wird die Kühlluft vom hinteren bis zum vorderen Ende des Gasentnahmerohres und zurückgeführt. Des Weiteren kann die Kühlluft im Kreislauf geführt werden, wobei die Temperatur der abgeführten Kühlluft vor dem erneuten Zuführen erhöht wird. Dabei kann auch vorgesehen werden, dass die Temperatur der abgeführten Kühlluft gemessen wird und ein Lufterhitzer in Abhängigkeit der gemessenen Temperatur derart angesteuert wird, dass die Temperatur der im Kreislauf geführten Kühlluft im Bereich der Zuführung am hinteren Ende des Gasentnahmerohres einen vorgegebenen Sollwert aufweist. Die Temperatur des zuzuführenden Kühlgases und deren Menge wird so eingestellt, dass das zu analysierende Gas vom vorderen bis zum hinteren Ende des Gasentnahmerohres höchstens bis zu einer Minimaltemperatur abgekühlt wird, die größer oder gleich der Taupunkttemperatur der im zu analysierenden Gas enthaltenen Komponenten ist. Weiterhin kann vorgesehen werden, dass am hinteren Ende des Gasentnahmerohrs die Temperatur der zugeführten Kühlluft höher als die Temperatur des zu analysierenden Gases und die Temperatur der abgeführten Kühlluft kleiner oder gleich der Temperatur des zu analysierenden Gases ist. Durch die Temperatur und Menge der Kühlluft wird das Temperaturprofil des Gasentnahmerohres über seine gesamte Länge derart eingestellt, dass die minimale Temperatur größer oder gleich der Taupunkttemperatur der im zu analysierenden Gas enthaltenen Komponenten ist. Je nach Temperatur des zu analysierenden Gases wird die Temperatur der abgeführten Kühlluft vor dem erneuten Zuführen um wenigstens 20°C, vorzugsweise um wenigstens 50°C, höchstvorzugsweise um wenigstens 75°C, erhöht. Die Temperatur der zugeführten Kühlluft im Bereich des hinteren Endes des Gasentnahmerohres wird vorzugsweise in einem Bereich von 100°C und 600°C eingestellt. Die Temperatur des zu analysierenden Gases im Prozessraum, also vor der Entnahme, kann in einem Bereich von 200°C bis 1600°C liegen. Die Gasentnahmesonde wird weiterhin mit Kühlluft derart betrieben, dass die Temperatur des entnommenen und zu analysierenden Gases vom vorderen zum hinteren Ende des Gasentnahmerohres um wenigstens 50%, vorzugsweise um wenigstens 60%, höchstvorzugsweise um wenigstens 70% abgekühlt wird. Weiterhin ist es zweckmäßig, die Temperatur der abgeführten Kühlluft kleiner oder gleich der Temperatur des Gasentnahmerohres im Bereich des hinteren Endes des Gasentnahmerohres zu halten.

Der Lufterhitzer steht zweckmäßigerweise mit einer Steuereinrichtung in Verbindung, die den Lufterhitzer in Abhängigkeit eines Temperatursignals einer Temperaturmesseinrichtung ansteuert, wobei die Temperaturmesseinrichtung die Temperatur einer über die Kühlluftabführöffung abgeführten Kühlluft erfasst. Gemäß einem ersten Ausführungsbeispiel der Gasentnahmesonde ist die zwischen Gasentnahmerohr und Außenmantel ausgebildete Kühlzone in zwei sich über die Länge des Gasentnahmerohres erstreckende Hälften aufgeteilt, die im vorderen Bereich des Gasentnahmerohres über einen Überströmbereich miteinander verbunden sind und die Kühlluftzuführöffnung und die Kühlluftabführöffnung jeweils an einer der beiden Hälften im hinteren Bereich des Gasentnahmerohres vorgesehen sind. Gemäß einem zweiten Ausführungsbeispiel weist die zwischen Gasentnahmerohr und Außenmantel ausgebildete Kühlzone zwei konzentrisch zueinander angeordnete Ringräume auf, die im vorderen Bereich des Gasentnahmerohres über einen Überströmbereich miteinander verbunden sind und die Kühlluftzuführöffnung und die Kühlluftabführöffnung jeweils an einen der beiden Ringräume im hinteren Bereich des Gasentnahmerohres angeschlossen sind.

Die maximale Temperatur des zu analysierenden Gases ist durch die Temperatur im Prozessraum, aus dem das Gas abgezogen wird, gegeben. Für den Anwendungsfall einer Gasanalyse im Einlaufbereich eines Ofens für die Zementklinkerherstellung liegt diese Temperatur bei etwa 1200°C. Bei der Entnahme des zu analysierenden Gases durch das Gasentnahmerohrmuss muss sichergestellt werden, dass das Gas nicht kondensiert. Die minimale Temperatur des analysierenden Gases wird daher durch den niedrigsten Taupunkt der gasförmigen Komponenten innerhalb des Gases festgelegt. Für den oben genannten Anwendungsfall bei der Gasanalyse im Einlaufbereich eines Ofens für die Zementklinkerherstellung liegt eine Gastemperatur von ca. 200°C oberhalb der zu erwartenden Taupunkte. Andererseits soll die Temperatur des Gasentnahmerohres möglichst hoch sein, um eine Ansatzbildung zu minimieren. Die maximale Wandtemperatur im Bereich des vorderen Endes des Gasentnahmerohres ist durch die gewünschte Zeitstandfestigkeit des verwendeten Materials bestimmt. Eine luftgekühlte Gasentnahmesonde kann mit wesentlich höheren Wandtemperaturen als flüssigkeitsgekühlte Gasentnahmerohre betrieben werden, sodass Temperaturen im vorderen Bereich von 500°C bis 600°C eingestellt werden können.

Die minimale Wandtemperatur entlang des Gasentnahmerohres sollte die niedrigste Taupunkttemperatur der Komponenten im zu analysierenden Gas nicht unterschreiten. Die Einstellung der minimalen Temperatur des entnommenen, zu analysierenden Gases und die Verteilung der Wärme entlang des Gasentnahmerohres wird im Wesentlich durch die Temperatur des zugeführten Kühlgases und die Kühlluftmenge bzw. Geschwindigkeit bestimmt und muss an die jeweiligen Gegebenheiten angepasst werden. Um eine optimale Verteilung der Wärme entlang des Gasentnahmerohres zu gewährleisten, ist die Kühlluft mit einer Geschwindigkeit innerhalb der Gasentnahmesonde zu führen, die so hoch ist, dass es zu einer turbulenten Strömung kommt.

Weitere Vorteile und Ausgestaltungen der Erfindung werden anhand der nachfolgenden Beschreibung und der Zeichnung näher erläutert.

### In der Zeichnung zeigen

- Fig. 1: eine Prinzipdarstellung einer Gasentnahmeeinrichtung,
- Fig. 2a: eine Längsschnittdarstellung einer Gasentnahmesonde gemäß einem ersten Ausführungsbeispiel,
- Fig.2b: eine Querschnittdarstellung längs der Linie G-G der Fig. 2a,
- Fig. 3a: eine Längsschnittdarstellung einer Gasentnahmesonde gemäß einem zweiten Ausführungsbeispiel,
- Fig. 3b: eine Querschnittdarstellung längs der Linie J-J der Fig. 3a,
- Fig. 4: eine Prinzipdarstellung einer Gasentnahmeeinrichtung mit einer Steuereinrichtung zur Ansteuerung des Lufterhitzers in Abhängigkeit eines Temperatursignals,
- Fig. 5: schematische Schnittdarstellung der Einbausituation der Gasentnahmesonde mit Darstellung des Temperaturprofils,
- Fig. 6: Diagramm zu Darstellung des Wärmeeintrags in die Gasentnahmesonde durch die Einbausituation,
- Fig. 7: Diagramm zu Darstellung des Wärmeeintrags auf das Gasentnahmerohr,
- Fig. 8: Diagramm zur Darstellung des Temperaturverlaufs des entnommenen Gases und der Wandtemperatur des Gasentnahmerohres entlang seiner Länge,
- Fig. 9: Diagramm zur Darstellung des Temperaturverlaufs der Kühlluft entlang des Gasentnahmerohres und
- Fig. 10: Detailansicht des vorderen Endes der Gasentnahmesonde.

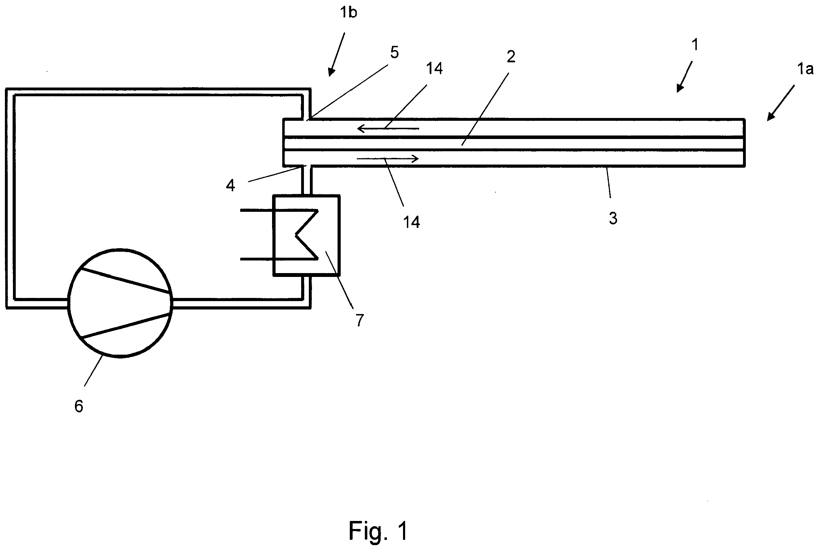

Die in Fig. 1 dargestellte Gasentnahmeeinrichtung weist eine Gasentnahmesonde 1 mit einem Gasentnahmerohr 2 auf, um ein zu analysierendes Gas im Bereich eines vorderen Endes 1a aus einem Prozessraum zu entnehmen und im Gasentnahmerohr bis zu einem hinteren Ende 1b zu führen. Das Gasentnahmerohr ist von einem Außenmantel 3 umgeben, wobei zwischen Gasentnahmerohr 2 und Außenmantel 3 Kühlluft 14 geführt wird, die am hinteren Ende 1b über eine Kühlluftzuführöffnung 4 zugeführt und über eine Kühlluftabführöffnung 5 abgeführt wird. Die Kühlluftabführöffnung und die Kühlluftzuführöffnung sind zur Ausbildung eines geschlossenen Kreislaufs miteinander verbunden, wobei dazwischen ein Ventilator 6 und ein Lufterhitzer 7 vorgesehen sind.

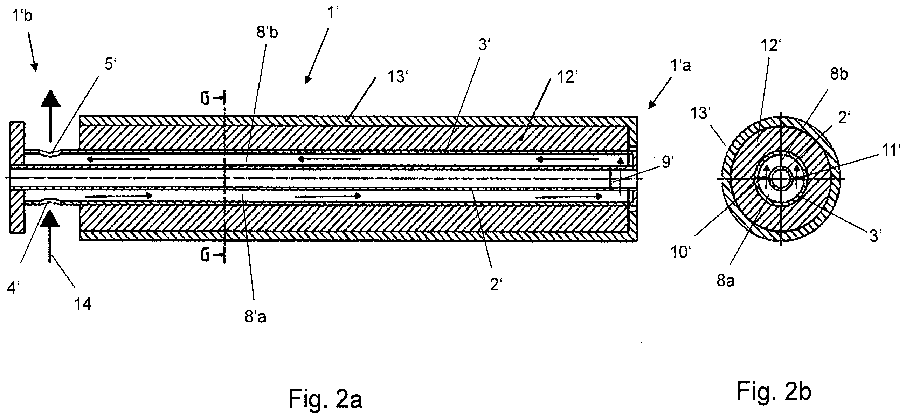

In den Fig. 2a und 2b ist eine Gasentnahmesonde 1' gemäß einem ersten Ausführungsbeispiel näher dargestellt, dort ist die zwischen Gasentnahmerohr 2' und Außenmantel 3' ausgebildete Kühlzone in zwei sich über die Länge des Gasentnahmerohres 2' erstreckende Hälften 8'a, 8'b unterteilt, die im vorderen Bereich 1'a der Gasentnahmesonde über einen Überströmbereich 9' miteinander verbunden sind. Die Kühlluftzuführöffnung 4' ist an die eine Hälfte 8'a und die Kühlluftabführöffnung 5' an die andere Hälfte 8'b im hinteren Bereich der Gasentnahmesonde angeschlossen. Die über die Kühlluftzuführöffnung 4' zugeführte Kühlluft 14 strömt somit vom hinteren Ende 1'b der Gasentnahmesonde 1' in der unteren Hälfte 8'a der Kühlzone bis zum vorderen Bereich 1'a und gelangt dort über den Überströmbereich 9' in die obere Hälfte 8'b der Kühlzone und strömt dort zurück zur Kühlluftabführöffnung 5'. Die Abgrenzung der beiden Hälften erfolgt dabei durch Trennwände 10', 11' (Fig. 2b).

Um die Gasentnahmesonde 1' vor einem übermäßigen Wärmeeintrag von außen, d. h. durch die Einbausituation, zu schützen, ist der Außenmantel 3' mit einer Isolierung 12' und einem Schutzrohr 13' umgeben. Die Strömung der über die Kühlluftzuführöffnung 4' zugeführten Kühlluft 14 ist innerhalb der Kühlzone mit Pfeilen dargestellt.

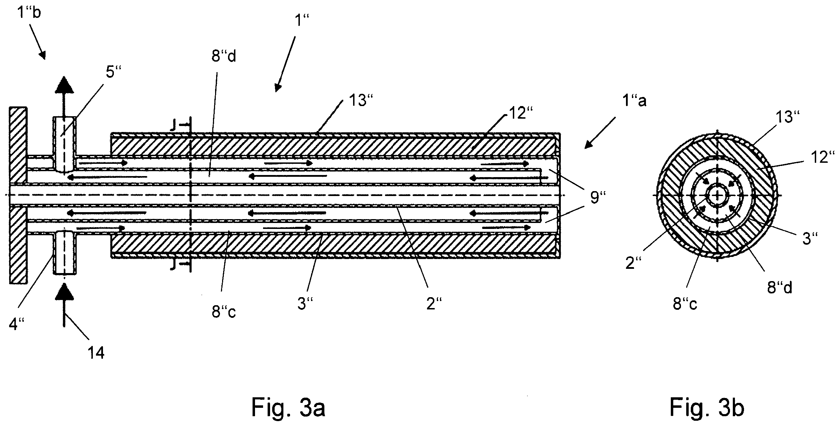

In den Fig. 3a und 3b ist eine Gasentnahmesonde 1" gemäß einem zweiten Ausführungsbeispiel offenbart, die sich im Wesentlichen nur durch die Ausbildung der Kühlzone unterscheidet. Die zwischen dem Gasentnahmerohr 2" und dem Außenmantel 3" ausgebildete Kühlzone wird hier durch zwei konzentrisch zueinander angeordnete Ringräume 8"c und 8"d gebildet, die im vorderen Bereich 1"a der Gasentnahmesonde 1" wiederum über einen Überströmbereich 9" miteinander verbunden sind. Die Kühlluftzuführöffnung 4" und die Kühlluftabführöffnung 5" sind jeweils an einen der beiden Ringräume 8"c, 8"d im hintern Bereich 1"b der Gasentnahmesonde 1" angeschlossen. Die Strömung der über die Kühlluftzuführöffnung 4" zugeführten Kühlluft 14 ist innerhalb der Kühlzone mit Pfeilen dargestellt.

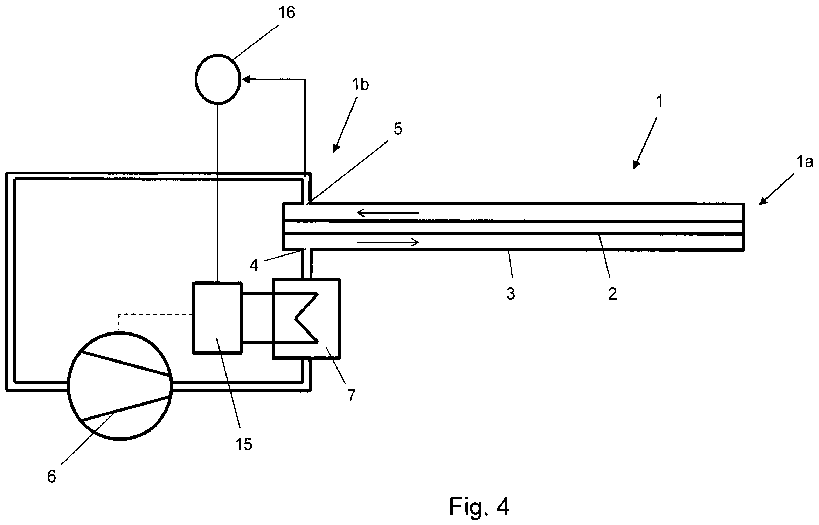

Fig. 4 zeigt die Gasentnahmeeinrichtung gemäß Fig. 1, die jedoch zusätzlich eine Steuereinrichtung 15 aufweist, die mit dem Lufterhitzer 7 in Verbindung steht und den Lufterhitzer in Abhängigkeit eines Temperatursignals einer Temperaturmesseinrichtung 16 ansteuert, wobei die Temperaturmesseinrichtung 16 die Temperatur einer über die Kühlluftzuführöffnung 5 abgeführten Kühlluft erfasst. Die Steuereinrichtung 15 kann ferner den Ventilator 6 ansteuern, um dadurch die Kühlluftmenge/Geschwindigkeit der Kühlluft zu regulieren.

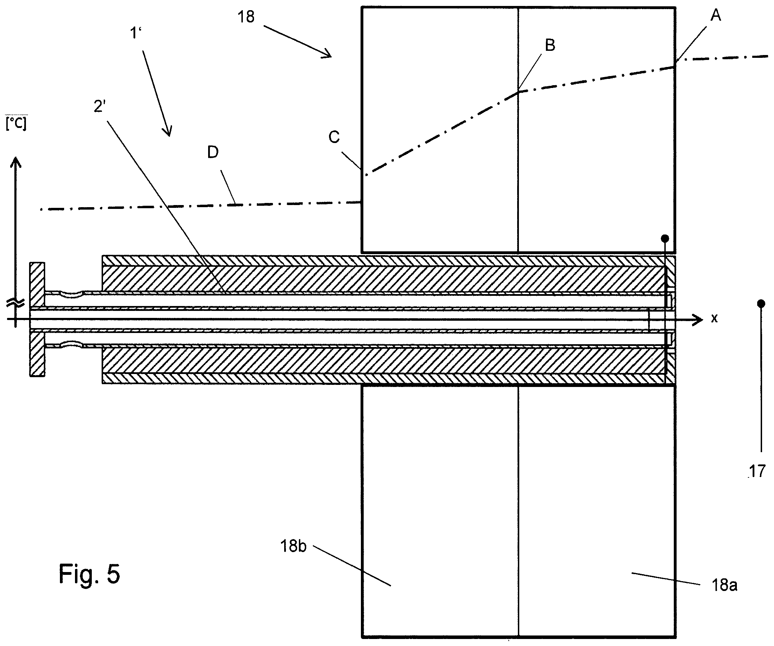

Fig. 5 zeigt die Gasentnahmesonde 1' der Fig. 2a in einer konkreten Einbausituation in einer, einen Prozessraum 17 umgebenden Wandung 18. Im dargstellten Ausführungsbeispiel mündet die Gasentnahmesonde 1' bündig mit der Wandung 18 in dem Prozessraum 17. Weiterhin ist der Temperaturverlaufs außerhalb der Gasentnahmesonde dargestellt. Die Wand ist im dargestellten Ausführungsbeispiel 2-lagig aufgebaut, wobei eine nach innen weisende feuerfeste Auskleidung 18a und eine Isolierung 18b und ggf. eine weitere Gehäusewand vorgesehen sind. Geht man von einer Situation aus, bei der das Gas im Prozessraum 17 etwa 1200°C heiß ist, beträgt die Temperatur der Wandung 18 an der Stelle A etwa 1100°C und am Punkt B etwa 960°C während sie an der Außenseite im Bereich des Punktes C nur noch ca. 200°C misst. Außerhalb der Wandung im Bereich D herrscht Umgebungstemperatur von beispielsweise 30°C.

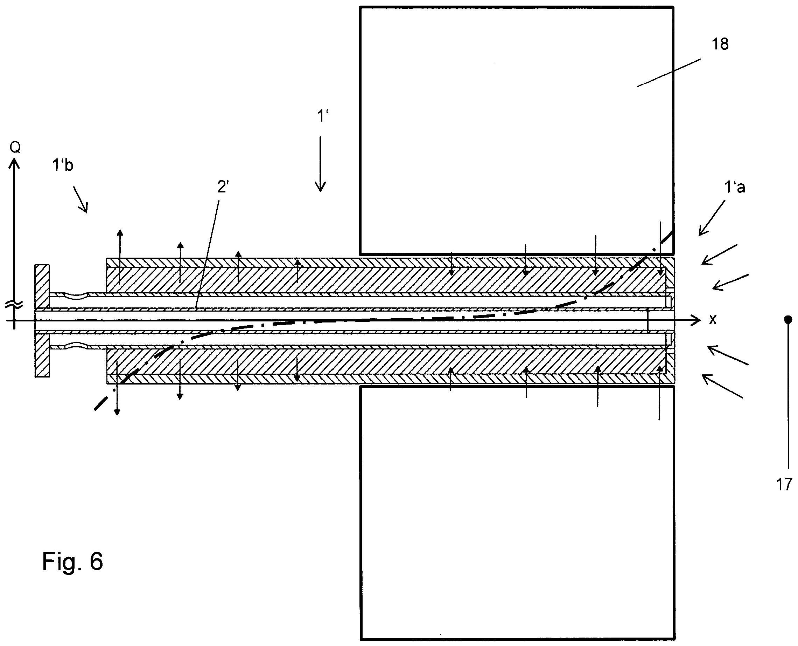

Fig. 6 zeigt ein Diagramm zur Darstellung des Wärmeeintrags in die Gasentnahmesonde durch die Einbausituation gemäß Fig. 5. Dabei wird insbesondere in den in der Wandung 18 steckenden, vorderen Teil der Gasentnahmesonde Wärme durch die heiße Umgebung (Wandung, Prozessraum) eingebracht (Wärmeaufnahmebereich), während der hintere Teil der Gasentnahmesonde durch den Kontakt mit der Umgebungsluft Wärmeenergie nach außen abgibt (Wärmeabstrahlungsbereich). Der Wärmeeintrag bzw. Wärmeaustrag wird durch Pfeile in diesen Bereichen symbolisiert.

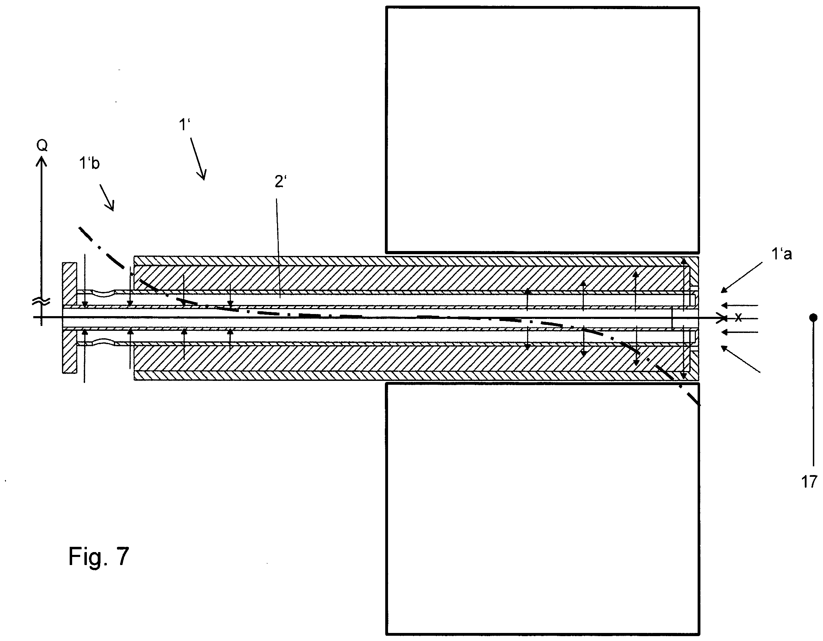

In Fig. 7 ist der Wärmeeintrag bzw. Wärmeaustrag bezüglich des Gasentnahmerohres 2' dargestellt, wobei das heiße Gasentnahmerohr im das vordere Ende 1'a aufweisenden, vorderen Bereich Wärmeenergie an das umgebende Kühlsystem, insbesondere die Kühlluft abgibt, während die Kühlluft im das hintere Ende 1'b aufweisenden, hinteren Bereich Wärmeenergie in das Gasentnahmerohr einbringt. Der Wärmeaustrag bzw. Wärmeeintrag wird wiederum durch Pfeile in diesen Bereich symbolisiert.

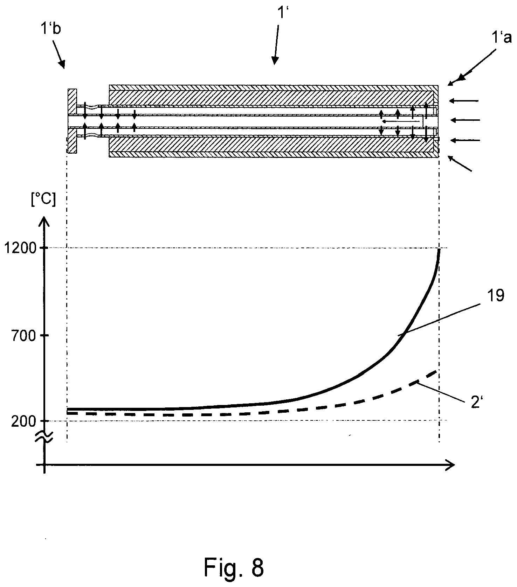

Der zugehörigen Temperaturverlauf des entnommen, zu analysierenden Gases 19 und die Temperatur des Gasentnahmerohres 2' ist aus Fig. 8 über die Länge der Gasentnahmesonde ersichtlich. Man erkennt, dass das zu analysierende Gas 19 von seiner Entnahmetemperatur bei etwa 1200°C auf ca. 200°C abgekühlt wird, während die Temperatur des Gasentnahmerohres am vorderen Ende 1'a ein deutlich niedrigeres Temperaturniveau aufweist und sich die Temperatur in Richtung zum hinteren Ende 1'b an die Temperatur des zu analysierenden Gases 19 anpasst.

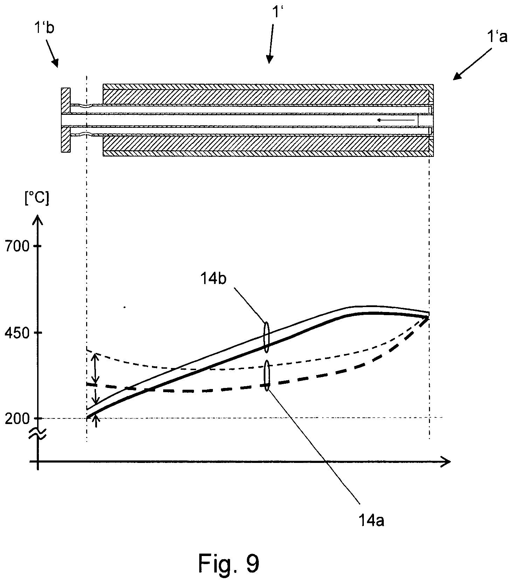

Parallel hierzu zeigt die Fig. 9 mit gestrichelten Linien den Vorlauftemperaturbereich 14a der Kühlluft und mit durchgezogenen Linien den Rücklauftemperaturbereich 14b der Kühlluft. Man sieht hier sehr deutlich, dass die Temperatur des Kühlgases im Bereich der Kühlluftzuführöffnung höher als im Bereich der Kühlluftabführöffnung ist. Diese sehr ungewöhnliche Temperaturverteilung wird dadurch erreicht, dass die Kühlluft zum einen die Wärme vom vorderen Bereich 1'a der Gasentnahmesonde zum hinteren Bereich 1'b verteilt und gleichzeitig das zu analysierende 19 abkühlt. Weiterhin strahlt die Gasentnahmesonde, insbesondere in den Bereichen, in denen sie nicht in der Wandung 18 eingebaut ist, nach außen ab. Die starke Kühlwirkung auf das zu analysierende Gas beruht vor allem auch darauf, dass etwa die 500-fache bis 2.500-fache Menge an Kühlluft im Vergleich zur Menge des zu analysierenden Gases zugeführt wird. Um die Wärme vom vorderen Bereich 1'a zum hinteren Bereich 1'b gut ableiten zu können, wird zweckmäßigerweise ein Material mit einer hohen Wärmeleitfähigkeit, beispielsweise Kohlenstoffnanoröhrchen, verwendet. Die Isolierung 12 dient vor allem dazu, dass keine zusätzliche Wärme von außen in die Gasentnahmesonde eingetragen wird. Um die Verteilung der Wärme entlang des Gasentnahmerohres zu verbessern, wird die Kühlluft 14 mit turbulenter Strömung in der Gasentnahmesonde geführt. Die erforderliche turbulente Strömung entsteht durch entsprechende Auswahl der Parameter "Fluidgeschwindigkeit" und "Viskosität", welche die für die Strömung charakteristische Reynoldszahl beeinflussen, sowie die Oberflächenbeschaffenheit der Rohrwandung. Des Weiteren könnte die Erzeugung der turbulenten Strömung durch eine entsprechend raue Oberfläche der die Kühlzone begrenzenden Wandungen unterstützt werden.

Über den Ventilator 6 und den Lufterhitzer 7 kann die Kühlluft in ihrer Geschwindigkeit/Menge und ihrer Temperatur an die äußeren Gegebenheiten angepasst werden, um einerseits eine zu starke Kühlung des zu analysierenden Gases unterhalb der Taupunkttemperatur der im zu analysierenden Gas enthaltenden Komponenten zu vermeiden und andererseits eine Vergleichsmäßigung des Temperaturprofils entlang der Gasentnahmesonde zu erreichen.

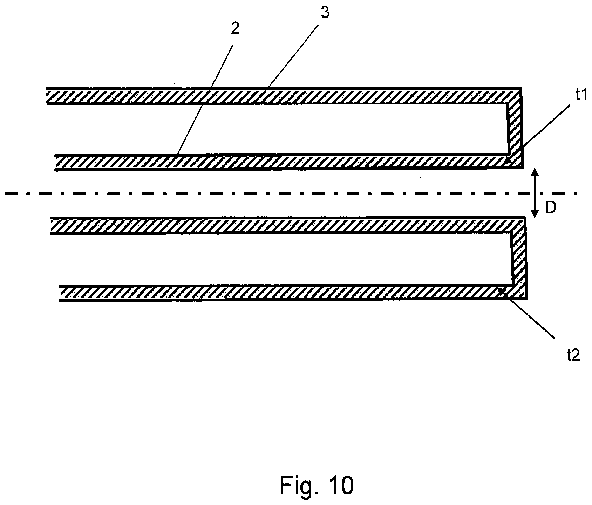

Mit Bezug auf Fig. 10 wird erläutert, wie beide Ziele erreicht werden können. Es hat sich als vorteilhaft erwiesen, wenn die Wandstärke t1 des Gasentnahmerohres 2 und die Wandstärke t2 des Außenmantels 3 bezüglich der Strömungsquerschnittsfläche des Gasentnahmerohres mit dem Innendurchmesser D so definiert wird, dass die Fläche, die sich aus den Kreisringflächen mit den Wandstärken t1 und t2 gebildet wird, größer oder gleich der 0,4-fachen Strömungsquerschnittsfläche des Gasentnahmerohres ist.

## Patentansprüche

1. Verfahren zum Betreiben einer Gasentnahmesonde (1), bei dem ein zu analysierendes Gas (19) im Bereich eines vorderen Endes (1a) eines Gasentnahmerohrs (2) aus einem Prozessraum (17) entnommen und im Gasentnahmerohr (2) bis zu einem hinteren Ende (1b) geführt und dabei gekühlt wird, indem zwischen dem Gasentnahmerohr (2) und wenigstens einem das Gasentnahmerohr umgebenden Außenmantel (3) Kühlluft (14) geführt wird, wobei die Kühlluft am hinteren Ende des Gasentnahmerohres zu- und abgeführt wird und die Temperatur des zu analysierenden Gases im Bereich des vorderen Endes des Gasentnahmerohrs höher als die Temperatur der zugeführten Kühlluft ist, und die Gasentnahmesonde nach außen abstrahlt,
**dadurch gekennzeichnet, dass** die Temperatur der zugeführten Kühlluft (14) höher als die Temperatur der abgeführten Kühlluft ist.

2. Verfahren nach Anspruch 1, **dadurch gekennzeichnet, dass** die Kühlluft vom hinteren bis zum vorderen Ende des Gasentnahmerohres (2) und zurückgeführt wird.

3. Verfahren nach Anspruch 1, **dadurch gekennzeichnet, dass** die Kühlluft (14) im Kreislauf geführt wird.

4. Verfahren nach Anspruch 1, **dadurch gekennzeichnet, dass** die Temperatur der abgeführten Kühlluft (14) vor dem erneuten Zuführen erhöht wird.

5. Verfahren nach Anspruch 3, **dadurch gekennzeichnet, dass** die Temperatur der abgeführten Kühlluft gemessen wird und ein Lufterhitzer (7) in Abhängigkeit der gemessenen Temperatur derart angesteuert wird, dass die Temperatur der im Kreislauf geführten Kühlluft im Bereich der Zuführung am hinteren Ende (1b) des Gasentnahmerohres (2) einen vorgegebenen Sollwert aufweist.

6. Verfahren nach Anspruch 1, **dadurch gekennzeichnet, dass** das zu analysierende Gas vom vorderen bis zum hinteren Ende des Gasentnahmerohres (2) höchstens bis zu einer Minimaltemperatur abgekühlt wird, die größer oder gleich der Taupunkttemperatur der im zu analysierenden Gas enthaltenen Komponenten ist.

7. Verfahren nach Anspruch 1, **dadurch gekennzeichnet, dass** am hinteren Ende (1b) des Gasentnahmerohrs (2) die Temperatur der zugeführten Kühlluft höher als die Temperatur des zu analysierenden Gases und die Temperatur der abgeführten Kühlluft kleiner oder gleich der Temperatur des zu analysierenden Gases ist.

8. Verfahren nach Anspruch 1, **dadurch gekennzeichnet, dass** das Temperaturprofil des Gasentnahmerohres (2) über seine gesamte Länge so eingestellt wird, dass die minimale Temperatur größer oder gleich der Taupunkttemperatur der im zu analysierenden Gas enthaltenen Komponenten ist.

9. Verfahren nach Anspruch 1, **dadurch gekennzeichnet, dass** die Temperatur der abgeführten Kühlluft vor dem erneuten Zuführen um wenigstens 20°C erhöht wird.

10. Verfahren nach Anspruch 1, **dadurch gekennzeichnet, dass** die Temperatur der zugeführten Kühlluft im Bereich des hinteren Endes (1b) des Gasentnahmerohrs (2) in einem Bereich von 100°C und 600°C eingestellt wird.

11. Verfahren nach Anspruch 1, **dadurch gekennzeichnet, dass** die Temperatur des zu analysierenden Gases vor der Entnahme in einem Bereich von 200°C bis 1600°C liegt.

12. Verfahren nach Anspruch 1, **dadurch gekennzeichnet, dass** die Temperatur des entnommenen und zu analysierenden Gases vom vorderen zum hinteren Ende des Gasentnahmerohres (2) um wenigstens 50% abgekühlt wird.

13. Verfahren nach Anspruch 1, **dadurch gekennzeichnet, dass** die Temperatur der abgeführten Kühlluft kleiner oder gleich der Temperatur des Gasentnahmerohrs (2) im Bereich des hinteren Endes des Gasentnahmerohres (2) ist.

14. Verfahren nach Anspruch 1, **dadurch gekennzeichnet, dass** die Gasentnahmesonde am Prozessraum derart angeordnet wird, dass sie in einem vorderen Bereich Wärme von außen aufnimmt und in einem hinteren Bereich Wärme nach außen abstrahlt, wobei in einer Gesamtwärmebilanz die Gasentnahmesonde mehr Wärme abstrahlt als aufnimmt.

15. Verfahren nach Anspruch 1, **dadurch gekennzeichnet, dass** der Differenzbetrag der aufgenommenen und abgestrahlten Wärme der Gasentnahmesonde der Summe der Kühlwärme des zu analysierendes Gases (19) und der Kühlluft (14) entspricht.

16. Gasentnahmeeinrichtung zur Durchführung des Verfahrens nach einem oder mehreren der vorhergehenden Ansprüche mit einer Gasentnahmesonde (1), die ein Gasentnahmerohr (2) aufweist, um ein zu analysierendes Gas im Bereich eines vorderen Endes (1a) zu entnehmen und im Gasentnahmerohr (2) bis zu einem hinteren Ende (1b) zu führen, wobei das Gasentnahmerohr (2) von wenigstens einem Außenmantel (3) umgeben ist, sodass eine sich über die Länge des Gasentnahmerohres (2) erstreckende Kühlzone ausgebildet ist, die im Bereich des hinteren Endes (1b) der Gasentnahmesonde (1) eine Kühlluftzuführöffnung zum Zuführen von Kühlluft und eine Kühlluftabführöffnung (5) zum Abführen von Kühlluft aufweist, wobei die Gasentnahmesonde nach außen abstrahlt,
**dadurch gekennzeichnet, dass** die Kühlluftabführöffnung (5) und die Kühlluftzuführöffnung (4) zur Ausbildung eines geschlossenen Kreislaufs miteinander verbunden sind und zwischen Kühlluftabführöffnung (5) und Kühlluftzuführöffnung (4) ein Lufterhitzer (7) zum Erhöhen der Temperatur der abgeführten Kühlluft vorgesehen ist.

17. Gasentnahmeeinrichtung nach Anspruch 16, **dadurch gekennzeichnet, dass** die Gasentnahmesonde derart am Prozessraum angeordnet ist, dass sie in einem vorderen Bereich einen Wärmeaufnahmebereich und in einem hinteren Bereich einen Wärmeabstrahlungsbereich ausbildet.

18. Gasentnahmeeinrichtung nach Anspruch 16, **dadurch gekennzeichnet, dass** der Lufterhitzer (7) mit einer Steuereinrichtung (15) in Verbindung steht, die den Lufterhitzer (7) in Abhängigkeit eines Temperatursignals einer Temperaturmesseinrichtung (16) ansteuert, wobei die Temperaturmesseinrichtung (16) die Temperatur einer über die Kühlluftabführöffnung (5) abgeführten Kühlluft erfasst.

19. Gasentnahmeeinrichtung nach Anspruch16, **dadurch gekennzeichnet, dass** die zwischen Gasentnahmerohr (2') und Außenmantel (3') ausgebildete Kühlzone in zwei sich über die Länge des Gasentnahmerohres erstreckende Hälften (8'a, 8'b) aufgeteilt ist, die im vorderen Bereich der Gasentnahmesonde (2') über einen Überströmbereich (9') miteinander verbunden sind und die Kühlluftzuführöffnung (4') und die Kühlluftabführöffnung (5') jeweils an einer der beiden Hälften im hinteren Bereich (1'b) der Gasentnahmesonde (1') vorgesehen sind.

20. Gasentnahmeeinrichtung nach Anspruch 16, **dadurch gekennzeichnet, dass** die zwischen Gasentnahmerohr (2") und Außenmantel (3") ausgebildete Kühlzone zwei konzentrisch zueinander angeordnete Ringräume (8"c, 8"d) aufweist, die im vorderen Bereich (1"a) der Gasentnahmesonde (1") über einen Überströmbereich (9") miteinander verbunden sind und die Kühlluftzuführöffnung (4") und die Kühlluftabführöffnung (5") jeweils an einen der beiden Ringräume (8"c; 8"d) im hinteren Bereich der Gasentnahmesonde vorgesehen sind.

## Claims

1. A method for operating a gas sampling probe (1), in which a gas (19) to be analyzed is removed from a process space (17) in the region of a front end (1a) of a gas sampling tube (2) and is conducted through the gas sampling tube (2) as far as a rear end (1b) and in the process is cooled in that cooling air (14) is conducted between the gas sampling tube (2) and at least one outer casing (3) enclosing the gas sampling tube, wherein the cooling air is fed and discharged at the rear end of the gas sampling tube and the temperature of the gas to be analyzed is higher in the region of the front end of the gas sampling tube than the temperature of the fed cooling air, and the gas sampling probe emits outward,
**characterized in that** the temperature of the fed cooling air (14) is higher than the temperature of the discharged cooling air.

2. The method as claimed in claim 1, **characterized in that** the cooling air is conducted from the rear to the front end of the gas sampling tube (2) and back again.

3. The method as claimed in claim 1, **characterized in that** the cooling air (14) is conducted in a circuit.

4. The method as claimed in claim 1, **characterized in that** the temperature of the discharged cooling air (14) is increased before it is fed again.

5. The method as claimed in claim 3, **characterized in that** the temperature of the discharged cooling air is measured and an air heater (7) is actuated depending on the temperature measured such that the temperature of the cooling air conducted in a circuit is at a prescribed setpoint value in the region of the feed at the rear end (1b) of the gas sampling tube (2).

6. The method as claimed in claim 1, **characterized in that** the gas to be analyzed is cooled from the front to the rear end of the gas sampling tube (2) at most to a minimum temperature that is greater than or equal to the dew point temperature of the components contained in the gas to be analyzed.

7. The method as claimed in claim 1, **characterized in that** the temperature of the fed cooling air is higher at the rear end (1b) of the gas sampling tube (2) than the temperature of the gas to be analyzed and the temperature of the discharged cooling air is less than or equal to the temperature of the gas to be analyzed.

8. The method as claimed in claim 1, **characterized in that** the temperature profile of the gas sampling tube (2) along its entire length is set such that the minimum temperature is greater than or equal to the dew point temperature of the components contained in the gas to be analyzed.

9. The method as claimed in claim 1, **characterized in that** the temperature of the discharged cooling air is increased by at least 20°C before it is fed again.

10. The method as claimed in claim 1, **characterized in that** the temperature of the fed cooling air is set in a range from 100°C to 600°C in the region of the rear end (1b) of the gas sampling tube (2).

11. The method as claimed in claim 1, **characterized in that** the temperature of the gas to be analyzed is in a range from 200°C to 1600°C prior to removal.

12. The method as claimed in claim 1, **characterized in that** the temperature of the gas removed and to be analyzed is cooled by at least 50% from the front to the rear end of the gas sampling tube (2).

13. The method as claimed in claim 1, **characterized in that** the temperature of the discharged cooling air is less than or equal to the temperature of the gas sampling tube (2) in the region of the rear end of the gas sampling tube (2).

14. The method as claimed in claim 1, **characterized in that** the gas sampling probe is arranged at the process space such that it absorbs heat from the outside in a front region and emits heat to the outside in a rear region, wherein, in an overall heat balance, the gas sampling probe emits more heat than it absorbs.

15. The method as claimed in claim 1, **characterized in that** the difference between the heat absorbed and emitted by the gas sampling probe corresponds to the sum of the cooling heat of the gas (19) to be analyzed and of the cooling air (14).

16. A gas sampling device for carrying out the method as claimed in one or more of the preceding claims, having a gas sampling probe (1) which has a gas sampling tube (2) in order to remove a gas to be analyzed in the region of a front end (1a) and to conduct it through the gas sampling tube (2) as far as a rear end (1b), wherein the gas sampling tube (2) is enclosed by at least one outer casing (3) such that a cooling zone that extends along the length of the gas sampling tube (2) is formed, said cooling zone having in the region of the rear end (1b) of the gas sampling probe (1) a cooling-air feed opening for supplying cooling air and a cooling-air discharge opening (5) for discharging cooling air, wherein the gas sampling probe emits outward,
**characterized in that** the cooling-air discharge opening (5) and the cooling-air feed opening (4) are connected together to form a closed circuit and an air heater (7) for raising the temperature of the discharged cooling air is provided between the cooling-air discharge opening (5) and the cooling-air feed opening (4).

17. The gas sampling device as claimed in claim 16, **characterized in that** the gas sampling probe is arranged at the process space such that it forms a heat absorption region in a front region and a heat emission region in a rear region.

18. The gas sampling device as claimed in claim 16, **characterized in that** the air heater (7) is connected to a control device (15) which actuates the air heater (7) in dependence on a temperature signal from a temperature measuring device (16), wherein the temperature measuring device (16) senses the temperature of cooling air discharged via the cooling-air discharge opening (5).

19. The gas sampling device as claimed in claim 16, **characterized in that** the cooling zone formed between the gas sampling tube (2') and the outer casing (3') is divided into two halves (8'a, 8'b) that extend along the length of the gas sampling tube, said halves being connected together in the front region of the gas sampling probe (2') via an overflow region (9') and the cooling-air feed opening (4') and the cooling-air discharge opening (5') each being provided on one of the two halves in the rear region (1'b) of the gas sampling probe (1').

20. The gas sampling device as claimed in claim 16, **characterized in that** the cooling zone formed between the gas sampling tube (2") and the outer casing (3") has two annular spaces (8"c, 8"d) that are arranged concentrically with one another, said annular spaces (8"c, 8"d) being connected together in the front region (1"a) of the gas sampling probe (1") via an overflow region (9"), and the cooling-air feed opening (4") and the cooling-air discharge opening (5") are each provided on one of the two annular spaces (8"c; 8"d) in the rear region of the gas sampling probe.

## Revendications

1. Procédé d'exploitation d'une sonde de prélèvement de gaz (1), selon lequel un gaz à analyser (19) est prélevé dans la zone d'une extrémité avant (1a) d'un tube de prélèvement de gaz (2) depuis une chambre de processus (17) et acheminé dans le tube de prélèvement de gaz (2) jusqu'à une extrémité arrière (1b) et est ainsi refroidi en faisant passer de l'air de refroidissement (14) entre le tube de prélèvement de gaz (2) et au moins une enveloppe extérieure (3) qui entoure le tube de prélèvement de gaz, l'air de refroidissement étant introduit et évacué au niveau de l'extrémité arrière du tube de prélèvement de gaz et la température du gaz à analyser dans la zone de l'extrémité avant du tube de prélèvement de gaz étant supérieure à la température de l'air de refroidissement introduit, et la sonde de prélèvement de gaz rayonnant vers l'extérieur,
**caractérisé en ce que** la température de l'air de refroidissement introduit (14) est supérieure à la température de l'air de refroidissement évacué.

2. Procédé selon la revendication 1, **caractérisé en ce que** l'air de refroidissement est acheminé depuis l'extrémité arrière jusqu'à l'extrémité avant du tube de prélèvement de gaz (2) et retour.

3. Procédé selon la revendication 1, **caractérisé en ce que** l'air de refroidissement (14) est acheminé en circuit.

4. Procédé selon la revendication 1, **caractérisé en ce que** la température de l'air de refroidissement évacué (14) est augmentée avant la nouvelle introduction.

5. Procédé selon la revendication 3, **caractérisé en ce que** la température de l'air de refroidissement évacué est mesurée et un dispositif de chauffage d'air (7) est commandé en fonction de la température mesurée de telle sorte que la température de l'air de refroidissement acheminé en circuit présente une valeur de consigne prédéfinie dans la zone de l'introduction à l'extrémité arrière (1b) du tube de prélèvement de gaz (2) .

6. Procédé selon la revendication 1, **caractérisé en ce que** le gaz à analyser est refroidi depuis l'extrémité avant jusqu'à l'extrémité arrière du tube de prélèvement de gaz (2) au plus jusqu'à une température minimale, qui est supérieure ou égale à la température de point de rosée des composants contenus dans le gaz à analyser.

7. Procédé selon la revendication 1, **caractérisé en ce qu'**à l'extrémité arrière (1b) du tube de prélèvement de gaz (2), la température de l'air de refroidissement introduit est supérieure à la température du gaz à analyser et la température de l'air de refroidissement évacué est inférieure ou égale à la température du gaz à analyser.

8. Procédé selon la revendication 1, **caractérisé en ce que** le profil de température du tube de prélèvement de gaz (2) sur l'ensemble de sa longueur est ajusté de telle sorte que la température minimale est supérieure ou égale à la température de point de rosée des composants contenus dans le gaz à analyser.

9. Procédé selon la revendication 1, **caractérisé en ce que** la température de l'air de refroidissement évacué est augmentée d'au moins 20 °C avant la nouvelle introduction.

10. Procédé selon la revendication 1, **caractérisé en ce que** la température de l'air de refroidissement introduit dans la zone de l'extrémité arrière (1b) du tube de prélèvement de gaz (2) est ajustée dans une plage allant de 100 °C à 600 °C.

11. Procédé selon la revendication 1, **caractérisé en ce que** la température du gaz à analyser se situe dans une plage allant de 200 °C à 1 600 °C avant le prélèvement.

12. Procédé selon la revendication 1, **caractérisé en ce que** la température du gaz prélevé et à analyser est refroidie d'au moins 50 % depuis l'extrémité avant jusqu'à l'extrémité arrière du tube de prélèvement de gaz (2).

13. Procédé selon la revendication 1, **caractérisé en ce que** la température de l'air de refroidissement évacué est inférieure ou égale à la température du tube de prélèvement de gaz (2) dans la zone de l'extrémité arrière du tube de prélèvement de gaz (2).

14. Procédé selon la revendication 1, **caractérisé en ce que** la sonde de prélèvement de gaz est agencée sur la chambre de processus de telle sorte qu'elle reçoit de la chaleur depuis l'extérieur dans une zone avant et rayonne de la chaleur vers l'extérieur dans une zone arrière, la sonde de prélèvement de gaz rayonnant plus de chaleur qu'elle n'en reçoit dans un bilan de chaleur global.

15. Procédé selon la revendication 1, **caractérisé en ce que** le montant de la différence entre la chaleur reçue et rayonnée de la sonde de prélèvement de gaz correspond à la somme de la chaleur de refroidissement du gaz à analyser (19) et de l'air de refroidissement (14) .

16. Appareil de prélèvement de gaz pour la réalisation du procédé selon une ou plusieurs des revendications précédentes, comprenant une sonde de prélèvement de gaz (1), qui comprend un tube de prélèvement de gaz (2), afin de prélever un gaz à analyser dans la zone d'une extrémité avant (1a) et de l'acheminer dans le tube de prélèvement de gaz (2) jusqu'à une extrémité arrière (1b), le tube de prélèvement de gaz (2) étant entouré par au moins une enveloppe extérieure (3), de telle sorte qu'une zone de refroidissement s'étendant sur la longueur du tube de prélèvement de gaz (2) est formée, qui comprend dans la zone de l'extrémité arrière (1b) de la sonde de prélèvement de gaz (1) une ouverture d'introduction d'air de refroidissement pour l'introduction d'air de refroidissement et une ouverture d'évacuation d'air de refroidissement (5) pour l'évacuation d'air de refroidissement, la sonde de prélèvement de gaz rayonnant vers l'extérieur, **caractérisé en ce que** l'ouverture d'évacuation d'air de refroidissement (5) et l'ouverture d'introduction d'air de refroidissement (4) sont reliées entre elles pour la formation d'un circuit fermé, et un dispositif de chauffage d'air (7) est prévu entre l'ouverture d'évacuation d'air de refroidissement (5) et l'ouverture d'introduction d'air de refroidissement (4) pour l'augmentation de la température de l'air de refroidissement évacué.

17. Appareil de prélèvement de gaz selon la revendication 16, **caractérisé en ce que** la sonde de prélèvement de gaz est agencée sur la chambre de processus de telle sorte qu'elle forme une zone de réception de chaleur dans une zone avant et une zone de rayonnement de chaleur dans une zone arrière.

18. Appareil de prélèvement de gaz selon la revendication 16, **caractérisé en ce que** le dispositif de chauffage d'air (7) est raccordé à un appareil de commande (15), qui commande le dispositif de chauffage d'air (7) en fonction d'un signal de température d'un appareil de mesure de température (16), l'appareil de mesure de température (16) détectant la température d'un air de refroidissement évacué par l'intermédiaire de l'ouverture d'évacuation d'air de refroidissement (5) .

19. Appareil de prélèvement de gaz selon la revendication 16, **caractérisé en ce que** la zone de refroidissement formée entre le tube de prélèvement de gaz (2') et l'enveloppe extérieure (3') est divisée en deux moitiés (8'a, 8'b) s'étendant sur la longueur du tube de prélèvement de gaz, qui sont reliées entre elles dans la zone avant de la sonde de prélèvement de gaz (2') par l'intermédiaire d'une zone de débordement (9'), et l'ouverture d'introduction d'air de refroidissement (4') et l'ouverture d'évacuation d'air de refroidissement (5') sont chacune prévues sur une des deux moitiés dans la zone arrière (1'b) de la sonde de prélèvement de gaz (1').

20. Appareil de prélèvement de gaz selon la revendication 16, **caractérisé en ce que** la zone de refroidissement formée entre le tube de prélèvement de gaz (2") et l'enveloppe extérieure (3") comprend deux chambres annulaires (8"c, 8"d) agencées concentriquement l'une par rapport à l'autre, qui sont reliées entre elles dans la zone avant (1"a) de la sonde de prélèvement de gaz (1") par l'intermédiaire d'une zone de débordement (9"), et l'ouverture d'introduction d'air de refroidissement (4") et l'ouverture d'évacuation d'air de refroidissement (5") sont chacune prévues sur une des deux chambres annulaires (8"c ; 8"d) dans la zone arrière de la sonde de prélèvement de gaz.
